# EUROPEAN PATENT SPECIFICATION

(11) **EP 2 393 516 B1**
(45) Date of publication and mention of the grant of the patent: **02.12.2015**
(21) Application number: 10707989.9
(22) Date of filing: 02.02.2010
(51) Int. Cl.: A61K 9/20, A61K 9/00, A61K 31/198, A61K 31/375, A61K 9/46

(54) **STABLE, TASTE AND ODOR MASKED PHARMACEUTICAL COMPOSITIONS COMPRISING N-ACETYLCYSTEINE AND VITAMIN C**
STABILE PHARMAZEUTISCHE ZUSAMMENSETZUNGEN MIT N-ACETYLCYSTEIN UND VITAMIN C MIT MASKIERTEM GESCHMACK UND GERUCH
COMPOSITIONS PHARMACEUTIQUES STABLES, À GOÛT ET ODEUR MASQUÉS COMPRENANT DE LA N-ACÉTYLCYSTÉINE ET DE LA VITAMINE C

(30) Priority: 05.02.2009 TR 200900881
(43) Date of publication of application: 14.12.2011
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000023
(87) International publication number: WO 2010/090612

(56) References cited:
- EP-A1- 0 349 797
- EP-A1- 0 351 353
- WO-A1-94/07477
- WO-A2-02/17921
- DE-A1- 4 103 360
- FR-A1- 2 333 494
- FR-A1- 2 913 338
- GB-A- 2 192 790
- US-A- 5 415 870
- US-A- 6 066 335
- US-A1- 2002 037 855
- US-A1- 2010 119 589
- ANONYMOUS ED - ROTE LISTE SERVICE GMBH (ED): "ROTE LISTE 2004, PASSAGE", 1 January 2004 (2004-01-01), ROTE LISTE(R) 2004 : ARZNEIMITTELVERZEICHNIS FÜR DEUTSCHLAND (EINSCHLIESSLICH EU-ZULASSUNGEN UND BESTIMMTER MEDIZINPRODUKTE; [ROTE LISTE], EDITIO VERLAG, AULENDORF, DE, PAGE(S) PARAGRAPH05001 - PARAGRAPH05033, XP002608589, ISBN: 978-3-87193-286-1

## Description

### Field of the invention

The present invention is related to the stable, taste and odor masked pharmaceutical compositions and their medical use.

### Background of the invention

The present invention provides a combination which is effective in the treatment of acute and chronic respiratory diseases and bronchial secretion disorders for removal and reduction of thick and viscous mucus and in cases where expectoration should be facilitated and for prevention of liver failure caused by high dose paracetamol intake. The effect caused by the combination prepared according to the present invention is hereinafter referred to as "desired effect". The combination disclosed herein includes N-Acetylcysteine (NAC) and Vitamin C.

NAC (formula I) is an acetylated derivative of the aminoacid L-cysteine which has a free thiol group (N-Acetyl-L-Cysteine).

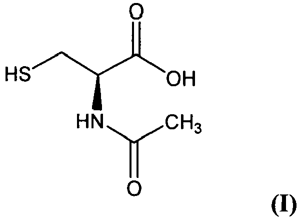

NAC is first described in patent US3184505 A. The patent discloses processes for the preparation of NAC, its mucolytic activity and its use in the treatment of respiratory diseases.

NAC, which is the ready to use form of L-cysteine, is more stable than L-cysteine and its absorption is higher than the absorption of L-cysteine. NAC has antioxidant activity as a reducing agent. Moreover, in addition to its liver protective and mucolytic activity, it has anti-apoptotic activity. Mucolytic activity of NAC is assumed to be related to the thiol groups' ability of reducing the disulphide bonds of the mucoproteins that are present in the mucus. With this effect; the viscosity of bronchial secretation is reduced and taking them out by cough reflex becomes easier. As a result breathing becomes easier. Moreover, NAC accelerates detoxication by the conversion into metabolites which are capable of stimulating glutation synthesis in the body and shows free radical scavenging effect. It is assumed that; NAC and glutathione's reduction of inflammation by neutralizing endogenous and exogenous oxidants is part of the mechanism of action.

Vitamin C (Formula II) is water soluble vitamin that is the L enantiomer of ascorbic acid.

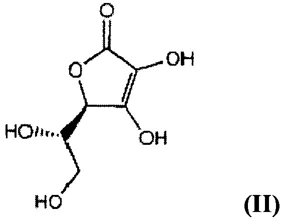

Vitamin C is an antioxidant that inhibits free radicals. Vitamin C is associated with tyrosine, carbohydrates, noradrenaline, histamine, phenylalanine and iron metabolism. Lipid, protein and carnitine synthesis; providing resistance to infections, serotonin hydroxylation; protection of integrity and functionality of blood vessels; cellular respiration; management of distribution and storage of iron; treatment of chronic iron toxicity; tissue repair and collagen production; conserving the healthy state of tissues; treatment of cold; flu; gum infections; acne and depression are some of the cases in which Vitamin C is strongly needed.

In studies carried on Vitamin C; a correlation between the use of Vitamin C and reduction in severity and duration of cold symptoms was found. (*"*Vitamin C supplementation and common cold symptoms: factors affecting the magnitude of the benefit", H. Hemilä, Medical Hypotheses (1999) 52(2), 171-178*).* The effect of Vitamin C on cold is thought to be the result of its reaction with oxidants (antioxidant activity). Because oxidants play an imprtant role on many diseases such as respiratory system infections, disease related to immune system, the toxicity caused by certain drugs, arteriosclerosis and cancer.

Synergistic effect of NAC and Vitamin C, has been proved in various clinical trials.
- Constamini D, Padoan R., Ann B Millar D., Valede A., Guadagni L. Synergic effect of vitamin C to oral N-acetylcysteine on mucus clearance. Curr. Ther. Res. (1986) 21, 6, 612-615.
- Bellomo G. Miranda Ribeiro T., Guidici, Alessandro Oliva S., Santos M. Treatment of bronchial diseases in pediatrics. Results of the use of oral acetylcysteine and vitamin C. Min. Ped. (1982) 17, 114-116.
- Kelly G. S. Attenuation of influenza-like symptomatology and improvement of cell-mediated immunity with long-term N-acetylcysteine and ascorbic acid treatment. Alt Med Rev. 1998; 5(3): 213-218.

When state of the art is taken into consideration; solutions in providing synergistic effect is directed to binary combinations and simultaneous use. However, more importantly, there is a strong need to combine active ingredients which have synergistic effect in a single dosage form.

According to research in developed countries, only 50% of the patients with chronic diseases, fulfill the requirements of the treatment. [Sabate, E. "Adherence to Long term Therapies: Evidence for Action". World Health Organization. Geneva, 2003. 212 pp. ISBN 92-4-154599-2]. In general, it was determined that 40-60% of patients do not take their medication as prescribed and this causes even worse consequences [Heneghan CJ, Glasziou P, Perera R The Cochrane Library, ISSN 1464-780X].

Patients skip taking prescribed drugs because of the reasons such as, forgetfulness, busy life, drugs in different time schedule, not being in an appropirate enviroment to get drug, misunderstanding of illness and treatment, communication problems with the doctor, inability of the doctor to control the patient's treatment, no confidence to doctor and medication, side effects, perspective to illness and medication, cognitive disorders, financial problems and complicated treatment regimens.

A complicated dosage regime, especially using multiple drugs several times a day is one of the most significant factor that prevents the patient's complience to treatment. As the treatment regimen becomes simpler, patient compliance and therefore the success of treatment will be higher. For example, the active ingredient combined in a single dosage form to simplify treatment regimens, will increase patient complience to treatment [Blonde L. Medication Compliance and Persistence With Therapy. Managed Care (2000). Volume 9, Number 9; archives.who.int/icium/icium2004/resources/ppt/AC016.ppt]. The use of a combination in a single dosage form will eliminate the negative effect on patient originating from using multiple drugs.

The present invention provides a combination of NAC as a mucolytic agent and Vitamin C as an antioxidant in a single dosage form; wherein the combination has desired effect which in turn provides complience of patient to treatment that eventually leads to increase in success of treatment.

Object of the present invention is to provide a formulation prepared according to the present invention that comprise pharmaceutically acceptable, non-toxic and therapeutically effective amounts of NAC and vitamin C and to combine these two active agents in a single dosage form.

However there remain some problems in the pharmaceutical compositions containing NAC. For example, since NAC is a highly acidic substance when it remains in the mouth for a long time, it causes irritation of the oral cavity and mucous membrane of the tongue. Moreover, due the presence of sulphur group in the molecule, taste and odor of NAC is extremely bad.

In patent numbered EP0339508 B1, a solution is disclosed for prevention of irritation of mucuous membrane and to mask the taste of NAC. The patent comprises a mouth soluble tablet composition wherein, the weight ratio of sodium bicarbonate and potassium bicarbonate is between 93:7 and 97:3 and wherein the said mixture is present in an amount of 45-55 parts per weight based on 100 parts by weight of acetylcysteine; a mixture of two carbohydrates selected from the group consisting of sorbitol xylitol and fructore, wherein the weight ratio between said two carbohydrates is from 0.8 to 1.2 and 1.2 to 0.8, and wherein said carbohydrate mixture is present in an amount of 20-50 parts by weight based on 1 part by weight of acetylcysteine and a fruit flavoring agent.

In EP0340662 B1, a water soluble pharmaceutical formulation comprising 5-25 parts by weight NAC, 70-85 parts xylitol, 0.5-1.2 parts sodium saccharin bihydrate, 0.4-1.2 part beta carotene and 4-7.5 parts flavoring agent is disclosed as a solution to mask the unpleasent taste and odor of NAC.

In patent numbered EP0481294 B2, orally administered, fast dissolving, solid drug preparations have been described as a solution to mask the unpleasent taste and odor of NAC. Said drug preparations consist of NAC whose proportion by the total weight of formulation is at least 50%, and the remaining amount of the preparation contains as inactive ingredients in relation to NAC by weight 15-25% cellulose and/or 4-12% cellulose derivatives, 25-35% sugar alcohol, 4-12% sweetener, 2-8%, flavoring and, if need be, 2-8% adjuvant that contain other auxiliary agent(s). As a result, in state of the art, the unpleasent taste and odor and its high rate of acidity is usually masked with use of sweeteners and flavoring agents in high amounts. However, using high amounts of these substances prevents using NAC in high amounts and limits the use of other substances which are essential for the formulation of active ingredients. However NAC can be used in a dose of up to 2500 mg. Using NAC in high amounts makes the taste and odor of the formulation worse. Present invention provides a taste and odor masked NAC composition without requiring the use of sweetener and flavoring agents in high amounts.

Vitamin C tends to degrade when exposed to heat, light, air, moisture, metal ions or an enviroment that has alkaline pH. It is unstable in aqueous solutions, and poorly soluble in nonaqueous solvents. Therefore, formulations containing vitamin C has stability problems. This brings the necessity of selecting the suitable excipient composition to provide the stability of Vitamin C in a tablet dosage form.

US2002/0037855 discloses effervescent tablets containing N-acetylcysteine and ascorbic acid.

The present invention provides a taste and odor masked stabilized pharmaceutical compositions wherein NAC and vitamin C are combined in a single dosage form so as to obtain desired effect starting from increasing patient's compliance and thus the success of the treatment. Pharmaceutical compositions prepared according to the present invention also includes at least one stabilizing agent to provide vitamin C stability; and at least one sweetener and/or flavoring agent in small amounts in order to contribute in taste and odor masking.

### Summary of the invention

The present invention relates to a pharmaceutical composition used in the manufacture of a drug which is effective in the treatment of acute and chronic respiratory diseases; bronchial secretion disorders; for removal and reduction of thick and viscous mucus and in cases where expectoration should be facilitated and for prevention of liver failure caused by high dose paracetamol intake characterized in that the said composition comprises a combination of therapeutically effective amount of NAC and therapeutically effective amount of vitamin C in a single dosage form and NaCl is present in the said composition as a taste regulating agent.

### Detailed description of the invention

The present invention provides pharmaceutical compsitions with desired effect, in which NAC and Vitamin C are formulated in a single dosage form in order to increase the complience of patient to treatment and thus increase the succes of the treatment. Present invention also allows using NAC in high amounts such as up to 2500mg and provides stable pharmaceutical compositions wherein the unpleasant taste and odor is masked.

According to the invention, pharmaceutical compositions in which NAC and Vitamin C are combined in a single dosage form, are effective in the treatment of several diseases and conditions and they increase patients' complience to treatment which in turn increases the success of treatment.

Another feature of the present invention is to formulate the combination of NAC and Vitamin C in certain amounts, sufficient amount of sodium chloride, sufficient amount of at least one sweetener/flavoring agent and optionally pharmaceutically acceptable excipients selected from stabilizing agent, diluent, binder, disintegrant, lubricant, glidant and surfactant formulated in a single dosage form. Said pharmaceutical compositions are masked without using sweetener and flavoring agents in high amounts.

Here, the term "several diseases and conditions" is related to; acute and chronic diseases of the respiratory diseases, bronchial secretion disorders, liver failure caused by high dose paracetamol intake, removal and reduction of thick and viscous mucus and facilitation of expectoration.

The terms "certain amount" and "sufficient amount" are related to; up to 70 % amount of NAC by weight, up to 20 % amount of Vitamin C by weight, up to 5% amount of sodium chloride, up to 5% amount of sweetener and/or flavoring agent and optionally excipients such as stabilizing agent, diluent, binder, disintegrant, lubricant, glidant ve surfactant are formulated in order to provide the desired therapeutic efficiency.

Pharmaceutically acceptable sweeteners can be selected from sucralose, sucrose, fructose, glucose, galactose, xylose, dextrose, levulose, lactose, maltose, maltodextrin, mannitol, maltitol, maltol, sorbitol, xylitol, erythritol, lactitol, isomalt, corn syrup, saccharin, saccharin salts, acesulfame potassium, aspartame, D-t ryptophan, monoammonium glycerssinate, neohesperidin dihydrochalcone, thaumatin, neotame, alitam, stevioside and cyclamate.

Pharmaceutically acceptable flavoring agents can be selected from natural aroma oil (e.g. peppermint oil, partridge currant oil, clove bud oil, parsley oil, eucalyptus oil, lemon oil, orange oil, etc.), menthol, mentane, anethole, methyl salicylate, eucalyptol, cinnamon, 1-methyl acetate, salvia, eugenol, oxanone, alpha-ionone, marjoram, lemon, orange, propenyl guaethol acetyl, sinnamon, vanilla, thymol, linaolol, cinnamaldehyde glycerol acetal, N-substituted p-menthane-3-carboxyamide, 3,1- methoxy propane 1,2-diol.

Stabilizing agent/agents can be selected from chelating agents and alkalizing agents. Stabilizing agent/agents can be present in the range of preferably 0-85% by weight, more preferably 0.1-75%.

Chelating agents can be selected from disodium EDTA, edetic acid, citric acid, sodium citrate, potassium citrate, or combinations thereof.

Alkalizing agents can be selected from alkali metal salts such as sodium carbonate, sodium hydrogen carbonate, sodium hydroxide, sodium silicate, disodium hydrogen orthophosphate, sodium aluminate; alkaline earth metal salts such as calcium carbonate, calcium hydroxide, dibasic calcium phosphate, tribasic calcium phosphate, calcium sulfate, calcium acetate, calcium gluconate, calcium glycerophosphate, magnesium carbonate, magnesium hydroxide, magnesium sulfate, magnesium acetate, magnesium silicate, magnesium aluminate; organic compounds such as primary, secondary and tertiary amines, cyclic amines, N, N'-dibenzyletylendiamine, diethanolamin, ethylenediamine, meglumine, monosodium glutamate, polacryline sodium, sodium alginate.

Pharmaceutically acceptable diluents can be selected from lactose, microcrystalline cellulose, starch, prejelatinized starch, modified starch, calcium phosphate (dibasic and/or tribasic), calcium sulfate trihydrate, calcium sulfate dihydrate, calcium carbonate, kaolin, lactitol, powdered cellulose, dextrose, dextrates, dextrin, sucrose, maltose, fructose, mannitol, sorbitol and xylitol. Diluents can be present in the range of preferably 0-85%, more preferably 0.1-75% by weight.

Pharmaceutically acceptable binders can be selected from starch (e.g.potato starch, corn starch, wheat starch, etc.), sucrose, glucose, dextrose, lactose, sugars such as maltodexstrin, natural and synthetic gums (such as acacia), gelatin, cellulose derivatives (microcrystalline cellulose, HPC, HEC, HPMC, carboxymethylcellulose, methylcellulose, ethylcellulose, etc.), polyvinylpyrrolidone (PVP), polyethylene glycol (PEG), waxes, calcium carbonate, calcium phosphate, alcohols (e.g. sorbitol, xylitol, mannitol) and water. Binder can be present in the range of preferably 0-10%, more preferably 0,1-5% by weight.

Pharmaceutically acceptable disintegrants can be selected from starch (corn starch, potato starch), sodium starch glycolate, pregelatinized starch, cellulose derivatives (e.g. croscarmellose sodium or microcrystalline cellulose), polyvinylpyrrolidone (PVP), crospovidone, alginic acid, sodium alginate, clays (xanthan gum or veegum etc.), ion-exchange resins and effervescent systems (alkali or alkaline earth metal carbonates [sodium carbonate, sodium hydrogen carbonate, potassium carbonate, potassium hydrogen carbonate, calcium carbonate, etc.]; water soluble polybasic organic acids and salts thereof such as sodium hydrogen sulfate, potassium hydrogen sulfate, sodium dihydrogen phosphate, succinic acid, tartaric acid, adipic acid, citric acid). Disintegrants can be present in the range of preferably 0-85%, more preferably 0.1-75% by weight.

Pharmaceutically acceptable lubricants can be selected from metallic stearates (magnesium stearate, calcium stearate, aluminum stearate, etc.), fatty acid esters (e.g. sodium stearyl fumarate), fatty acids (e.g. stearic acid), fatty alcohols, glyceryl behenate, mineral oil, paraffines, hydrogenated vegetable oils, leucine, polyethylene glycols (PEG), metallic lauryl sulfates (sodium lauryl sulfate, magnesium lauryl sulfate, etc.), sodium chloride, sodium benzoate, sodium acetate and talc. Lubricants can be present in the range of preferably 0-10%, more preferably 0.1-5% by weight.

Pharmaceutically acceptable glidants can be selected from silicon dioxide, magnesium trisilicate, powdered cellulose, starch, talc, tribasic calcium phosphate, metallic stearates, calcium silicate and metallic lauryl sulfate. Glidant can be present in the pharmaceutical formulation in proportions less than 1% by weight.

Pharmaceutically acceptable surfactants can be selected from polyoxyethlene-sorbitan-fatty acid esters (polysorbates), sodium lauryl sulfate, sodium stearyl fumarate, polyoxyethylene alkyl ethers, sorbitan fatty acid esters, polyethylene glycols (PEG), polyoxyethylene hint oil derivatives, docusate sodium, quaternary ammonium compounds, amino acids such as L-leucine, sugar esters of fatty acids and glycerides of fatty acids. Surfactant can be present in the range of preferably 0-10%, more preferably 0.1-5% by weight.

Additionally, pharmaceutically acceptable other excipients such as solubility modulators, electrolytes, colorants and coatings can be present in the formulation.

Pharmaceutical acceptable dosage forms are tablet, capsule, fast dissolving tablet, effervescent tablet, effervescent granule, fast dissolving granule, fast dissolving powder mixture, or dry powder form to prepare syrup.

Examples of pharmaceutical formulations of the invention are given below. These examples are given to illustrate the invention; the invention is not limited to disclosed examples.

### EXAMPLES

### Example 1.

| **Content** | **Amount (mg)** |
|---|---|
| NAC | 200 |
| Vitamin C | 100 |
| Sodium Chloride | 25 |
| PEG 6000 | 40 |
| PVP K-30 | 65 |
| Aspartame | 20 |
| Citric acid anhydride | 650 |
| Sodium hydrogen carbonate | 480 |
| Lemon flavor | 20 |
| **Tablet weight** | 1600 |

### Example 2.

| **Content** | **Amount (mg)** |
|---|---|
| NAC | 600 |
| Vitamin C | 200 |
| Sodium Chloride | 60 |
| PEG 6000 | 40 |
| PVP K-30 | 85 |
| Aspartame | 20 |
| Citric acid anhydride | 1490 |
| Sodium hydrogen carbonate | 1065 |
| Lemon flavor | 20 |
| **Tablet weight** | 3580 |

### Example 3.

| **Content** | **Amount (mg)** |
|---|---|
| NAC | 900 |
| Vitamin C | 400 |
| Sodium chloride | 75 |
| PEG 6000 | 45 |
| PVP K-30 | 85 |
| Aspartame | 30 |
| Citric acid anhydride | 1330 |
| Sodium hydrogen carbonate | 1135 |
| Lemon flavor | 30 |
| **Tablet weight** | 4030 |

### Example 4.

| **Content** | **Amount (mg)** |
|---|---|
| NAC | 1200 |
| Vitamin C | 400 |
| Sodium chloride | 80 |
| PEG 6000 | 45 |
| PVP K-30 | 85 |
| Aspartame | 30 |
| Citric acid anhydride | 1240 |
| Sodium hydrogen carbonate | 1090 |
| Lemon flavor | 30 |
| **Tablet weight** | 4200 |

## Claims

1. A pharmaceutical composition comprising, with respect to the core weight of the composition,
• NAC at a ratio up to 70% by weight
• Vitamin C at a ratio up to 20% by weight
• Sodium chloride at a ratio up to 5% by weight
• At least one sweetener and/or flavoring agent at a ratio up to 5% by weight, and
• Optionally one or more pharmaceutically acceptable excipients selected from stabilizing agent, diluent, binder, disintegrant, lubricant, glidant and surfactant,
wherein said composition is in the dosage forms of a tablet, capsule, fast dissolving tablet or effervescent tablet.

2. The composition according to claim 1, **characterized in that** the pharmaceutical composition contains NAC in doses up to 2500 mg.

3. The composition according to claim 1 **characterized in that**, sweeteners are selected from sucralose, sucrose, fructose, glucose, galactose, xylose, dextrose, levulose, lactose, maltose, maltodextrin, mannitol, maltitol, maltol, sorbitol, xylitol, erythritol, laktitol, isomalt, corn syrup, saccharin, saccharin salts, asesulfame potassium, aspartame, D-tryptophan, monoammonium glycerrisate, neohesperidin dihydrochalcone, thaumatin, neotame, alitam, stevioside and cyclamate.

4. The composition according to claim 1 **characterized in that**, flavoring agents are selected from natural aroma oils (e.g. peppermint oil, Partridge currant oil, clove bud oil, parsley oil, eucalyptus oil, lemon oil, orange oil, etc.), menthol, menta, anethole, methyl salicylate, eucalyptol, cinnamon, 1-methyl acetate, salvia, eugenol, oxanone, alpha-irison, marjoram, lemon, orange, propeny guaethol acetyl, sinnamon, vanilla, thymol, linaolol, cinnamaldehyde glycerol acetal, N-substituted-p-menthane-3-carboxyamide, 3,1-methoxy propane 1,2-diol.

5. The composition according to claim 1 **characterized in that**, pharmaceutical compositions contains chelating agents such as EDTA, edetic acid, citric acid, sodium citrate, potassium citrate, or combinations thereof as stabilizing agents.

6. The composition according to claim 1 **characterized in that**, the pharmaceutical composition comprise alkalizing agents, selected from alkali metal salts such as sodium carbonate, sodium hydrogen carbonate, sodium hydroxide, sodium silicate, disodium hydrogen orthophosphate, sodium aluminate; alkaline earth metal salts such as calcium carbonate, calcium hydroxide, dibasic calcium phosphate, tribasic calcium phosphate, calcium sulfate, calcium acetate, calcium gluconate, calcium glycerophosphate, magnesium carbonate, magnesium hydroxide, magnesium sulfate, magnesium acetate, magnesium silicate, magnesium aluminate; organic compounds such as primary, secondary and tertiary amines, cyclic amines, N, N'-dibenzyletylendiamine, diethanolamin, ethylenediamine, meglumine, Monosodium glutamate, polacryline sodium, sodium alginate as stabilizing agent.

7. The composition according to claims 5 and 6 **characterized in that**, stabilizing agent is in the range of preferably 0-85%, more preferably 0.1-75% by weight.

8. The composition according to claim 1, **characterized in that** diluent is selected from lactose, microcrystalline cellulose, starch, prejelatinized starch, modified starch, calcium phosphate (dibasic and/or tribasic), calcium sulfate trihydrate, calcium sulfate dihydrate, calcium carbonate, kaolin, lactitol, powdered cellulose, dextrose, dextrates, dextrin, sucrose, maltose, fructose, mannitol, sorbitol and xylitol, and is present in the range of preferably 0-85%, more preferably 0.1-75% by weight.

9. The composition according to claim 1 **characterized in that**, binder is selected from starch (e.g.potato starch, corn starch, wheat starch, etc.), sucrose, glucose, dextrose, lactose, sugars such as maltodextrin, natural and synthetic gums (such as acacia), gelatin, cellulose derivatives (microcrystalline cellulose, HPC, HEC, HPMC, carboxymethylcellulose, methylcellulose, ethylcellulose, etc.), polyvinylpyrrolidone (PVP), polyethylene glycol (PEG), waxes, calcium carbonate, calcium phosphate, alcohols (e.g. sorbitol, xylitol, mannitol) and water, and is present in the range of preferably 0-10%, more preferably 0,1-5% by weight.

10. The composition according to claim 1 **characterized in that**, disintegrants selected from starch (corn starch, potato starch), sodium starch glycolate, pregelatinized starch, cellulose derivatives (e.g. croscarmellose sodium or microcrystalline cellulose), polyvinylpyrrolidone (PVP), crospovidone, alginic acid, sodium alginate, clays (xanthan gum or veegum etc.), ion-exchange resins and effervescent systems (alkali or alkaline earth metal carbonates [sodium carbonate, sodium hydrogen carbonate, potassium carbonate, potassium hydrogen carbonate, calcium carbonate, etc.], water soluble polybasic organic acids and salts thereof such as sodium hydrogen sulfate, potassium hydrogen sulfate, sodium dihydrogen phosphate, succinic acid, tartaric acid, adipic acid, citric acid), and is present in the range of preferably 0-85%, more preferably 0.1-75% by weight.

11. The composition according to claim 1 **characterized in that**, lubricant is selected from metallic stearates (magnesium stearate, calcium stearate, aluminum stearate, etc.), fatty acid esters (e.g. sodium stearyl fumarate), fatty acids (e.g. stearic acid), fatty alcohols, glyceryl behenate, mineral oil, paraffines, hydrogenated vegetable oils, leucine, polyethylene glycols (PEG), metallic lauryl sulfates (sodium lauryl sulfate, magnesium lauryl sulfate, etc.), sodium chloride, sodium benzoate, sodium acetate and talc, and is present in the range of preferably 0-10%, more preferably 0.1-5% by weight.

12. The composition according to claim 1 **characterized in that**, glidant is selected from silicon dioxide, magnesium trisilicate, powdered cellulose, starch, talc, tribasic calcium phosphate, metallic stearates, calcium silicate and metallic lauryl sulfate, and is present in an amount of less than 1% by weight.

13. The composition according to claim 1 **characterized in that**, surfactant is selected from polyoxyethlene-sorbitan-fatty acid esters (polysorbates), sodium lauryl sulfate, sodium stearyl fumarate, polyoxyethylene alkyl ethers, sorbitan fatty acid esters, polyethylene glycols (PEG), polyoxyethylene hint oil derivatives, docusate sodium, quaternary ammonium compounds, amino acids such as L-leucine, sugar esters of fatty acids and glycerides of fatty acids, and is present in the range of preferably 0-10%, more preferably 0.1-5% by weight.

14. The composition according to the any preceding claim **characterized in that**, dosage form comprises a pharmaceutical composition given below;
NAC at a ratio up to 70% by weight
Vitamin C at a ratio up to 20% by weight
Sodium chloride at a ratio up to 5% by weight
Citric acid anhydride at a ratio 0-85% by weight
Sodium hydrogen carbonate at a ratio 0-85% by weight
Aspartame and/or lemon flavor at a ratio up to 5% by weight
PEG at a ratio 0-10% by weight; and
PVP at a ratio 0-10% by weight.

## Patentansprüche

1. Eine pharmazeutische Zusammensetzung, die in Bezug auf das Kerngewicht der Zusammensetzung die Folgendes beinhaltet;
• NAC im Verhältnis bis zu %70 nach Gewicht
• Vitamin C im Verhältnis bis zu %20 nach Gewicht
• Natriumchlorid im Verhältnis bis zu %5 nach Gewicht
• Mindestens ein Süßungsmittel und/oder Aromastoff im Verhältnis bis zu %5 nach Gewicht, und
• Gegebenenfalls einen oder mehreren pharmazeutisch annehmbaren Hilfsstoffen, die aus Stabilisierungsmittel, Verdünnungsmittel, Bindemittel, Sprengmittel, Gleitmittel, Fließregulierungsmittel und Tensid ausgewählt werden, wobei die erwähnte Zusammensetzung in den Darreichungsformen von Tabletten, Kapseln, schnell löslichen Tabletten oder Brausetabletten ist.

2. Die Zusammensetzung nach Anspruch 1, **dadurch gekennzeichnet, dass** die pharmazeutische Zusammensetzung NAC in Dosen bis zu 2500 mg beinhaltet.

3. Die Zusammensetzung nach Anspruch 1, **dadurch gekennzeichnet, dass** Süßungsmittel aus Sucralose, Saccharose, Fructose, Glucose, Galactose, Xylose, Dextrose, Levulose, Lactose, Maltose, Maltodextrin, Mannit, Maltit, Maltol, Sorbitol, Xylitol, Erythritol, Laktitol, Isomalt, Maissirup, Saccharin, Saccharin-Salze, Acesulfam-Kalium, Aspartam, D-Tryptophan, Monoammonium glycerrisate, Neohesperidindihydrochalkon, Thaumatin, Neotam, Alitam, Steviosid und Cyclamat ausgewählt werden.

4. Die Zusammensetzung nach Anspruch 1, **dadurch gekennzeichnet, dass** Aromastoffe aus natürlichen Aromaölen (z.B Pfefferminzöl, Rebhuhn-Johannisbeeröl, Nelkenknospenöl, Petersilienöl, Eukalyptusöl, Zitronenöl, Orangenöl, usw.), Menthol, Menta, Anethol, Methylsalicylat, Eucalyptol, Zimt, 1-Methylacetat, Salbei, Eugenol, Oxanone, alpha-irison, Majoran, Zitrone, Orange, propeny guaethol Acetyl, Sinnamon, Vanille, Thymol, linaolol, Zimtaldehyd Glycerin Acetal, N-substituierte-p-Menthan-3-Carboxyamid, 3,1- MethoxyPropan 1,2-diol ausgewählt werden.

5. Die Zusammensetzung nach Anspruch 1, **dadurch gekennzeichnet, dass** die pharmazeutischen Zusammensetzungen Chelatbildner wie EDTA, Edetinsäure, Citronensäure, Natriumcitrat, Kaliumcitrat, oder Kombinationen davon als Stabilisierungsmittel beinhalten.

6. Die Zusammensetzung nach Anspruch 1, **dadurch gekennzeichnet, dass** die pharmazeutische Zusammensetzung Alkalisierungsmittel aus Alkalimetallsalzen wie Natriumcarbonat, Natriumhydrogencarbonat,Natriumhydroxid,Natriumsilikat,Dinatriumhydrogenorthophosphat, Natriumaluminat;Erdalkalimetallsalze wie Calciumcarbonat, Calciumhydroxid, zweibasisches Calciumphosphat,tribasisches Calciumphosphat, Calciumsulfat, Calciumacetat, Calciumgluconat, Calciumglycerophosphat, Magnesiumcarbonat, Magnesiumhydroxid, Magnesiumsulfat, Magnesiumacetat, Magnesiumsilikat, Magnesiumaluminat; organische Verbindungen wie primäre, sekundäre und tertiäre Aminen, cyclische Aminen, N, N'-dibenzyletylendiamine, Diethanolamin, Ethylendiamin, Meglumin, Mononatriumglutamat, polacryline Natrium, Natriumalginat als Stabilisierungsmittel beinhaltet.

7. Die Zusammensetzung nach den Ansprüchen 5 und 6, **dadurch gekennzeichnet, dass** das Stabilisierungsmittel im Bereich von vorzugsweise %0-85, mehr bevorzugt %0,1-75 nach Gewicht liegt.

8. Die Zusammensetzung nach Anspruch 1, **dadurch gekennzeichnet, dass** das Verdünnungsmittel aus Lactose, mikrokristalliner Cellulose, Stärke, zuvor verkleisterter Stärke, modifizierter Stärke, Calciumphosphat (zweibasisches und/oder dreibasisches), Calciumsulfat-Trihydrat, Calciumsulfat-Dihydrat, Calciumcarbonat, Kaolin, Lactit, pulverisierter Cellulose, Dextrose, Dextrate, Dextrin, Saccharose, Maltose, Fructose, Mannit, Sorbit und Xylit ausgewählt wird und im Bereich von vorzugsweise %0-85, mehr bevorzugt %0,1-75 nach Gewicht liegt.

9. Die Zusammensetzung nach Anspruch 1, **dadurch gekennzeichnet, dass** das Bindemittel aus Stärke (z.B Kartoffelstärke, Maisstärke, Weizenstärke, etc.), Saccharose, Glucose, Dextrose, Lactose, Zucker wie Maltodextrin, natürliche und synthetische Gummis (wie Gummi arabicum), Gelatine, Cellulosederivaten (mikrokristalline Cellulose, HPC, HEC, HPMC, Carboxymethylcellulose, Methylcellulose, Ethylcellulose, usw.), Polyvinylpyrrolidon (PVP), Polyethylenglykol (PEG), Wachse, Calciumcarbonat, Calciumphosphat, Alkoholen (z.B Sorbit Xylit, Mannit) und Wasser ausgewählt wird und im Bereich von vorzugsweise %0-10, mehr bevorzugt %0,1-5 nach Gewicht liegt.

10. Die Zusammensetzung nach Anspruch 1, **dadurch gekennzeichnet, dass** Sprengmittel aus Stärke (Maisstärke, Kartoffelstärke), Natriumstärkeglycolat, Quellstärke , Cellulosederivaten (zum Beispiel Croscarmellose-Natrium oder mikrokristalline Cellulose), Polyvinylpyrrolidon (PVP), Crospovidone, Alginsäure, Natriumalginat, Tone (Xanthangummi oder Veegum usw.), Ionenaustauschharze und Brausesysteme (Alkali- oder Erdalkalicarbonate [Natriumcarbonat, Natriumhydrogencarbonat, Kaliumcarbonat, Kaliumhydrogencarbonat, Calciumcarbonat, usw.], Wasserlöslichen polybasischen organischen Säuren und Salze davon wie Natriumhydrogensulfat , Kaliumhydrogensulfat, Natriumdihydrogenphosphat, Bernsteinsäure, Weinsäure, Adipinsäure, Zitronensäure) ausgewählt werden und im Bereich von vorzugsweise %0-85, mehr bevorzugt %0,1 -75 nach Gewicht liegen.

11. Die Zusammensetzung nach Anspruch 1, **dadurch gekennzeichnet, dass** das Gleitmittel aus Metallstearaten (Magnesiumstearat, Calciumstearat, Aluminiumstearat, usw.), Fettsäureester (z.B Natriumstearylfumarat), Fettsäuren (z.B Stearinsäure), Fettalkoholen, Glycerylbehenat, Mineralöl, Paraffinen, hydrierten Pflanzenölen, Leucin, Polyethylenglykole (PEG), metallischen Laurylsulfaten (Natriumlaurylsulfat, Magnesiumlaurylsulfat, usw.), Natriumchlorid, Natriumbenzoat, Natriumacetat und Talkum ausgewählt wird und in im Bereich von vorzugsweise %0-10, mehr bevorzugt %0,1-5 nach Gewicht liegt.

12. Die Zusammensetzung nach Anspruch 1, **dadurch gekennzeichnet, dass** das Fließregulierungsmittel aus Siliziumdioxid, Magnesiumtrisilikat, pulverisierter Cellulose, Stärke, Talkum, Tricalciumphosphat, Metallstearaten, Calciumsilikat und metallischem Laurylsulfat ausgewählt wird und in einer Menge von weniger als % 1 nach Gewicht vorhanden ist.

13. Die Zusammensetzung nach Anspruch 1, **dadurch gekennzeichnet, dass** Tensid aus Polyoxyethylen-Sorbitan-Fettsäureestern (Polysorbate), Natriumlaurylsulfat, Natriumstearylfumarat, Polyoxyethylenalkylether, Sorbitanfettsäureestern, Polyethylenglykolen (PEG), Polyoxyethylenkastorölderivaten, Docusatnatrium, quaternäre Ammoniumverbindungen, Aminosäuren wie L-Leucin, Zuckerester von Fettsäuren und Glyceride von Fettsäuren ausgewählt wird und im Bereich von vorzugsweise %0-10, mehr bevorzugt %0,1-5 nach Gewicht liegt.

14. Die Zusammensetzung nach einem der vorhergehenden Ansprüche und **dadurch gekennzeichnet, dass** Darreichungsform eine nachstehend angegebene pharmazeutische Zusammensetzung beinhaltet;
NAC im Verhältnis bis zu %70 nach Gewicht
Vitamin C im Verhältnis bis zu %20 nach Gewicht
Natriumchlorid im Verhältnis bis zu %5 nach Gewicht
Citronensäure-Anhydrid im Verhältnis von %0-85 nach Gewicht
Natriumhydrogencarbonat im Verhältnis von %0-85 nach Gewicht
Aspartam und/oder Zitronengeschmack im Verhältnis bis zu %5 nach Gewicht
PEG im Verhältnis von %0-10 nach Gewicht; und
PVP im Verhältnis von %0-10 nach Gewicht.

## Revendications

1. Une composition pharmaceutique comprenant par rapport au poids de base de la composition,
• CNA à un taux jusqu'à 70% en poids
• La vitamine C à un taux jusqu'à 20% en poids
• Chlorure de sodium à un taux jusqu'à 5% en poids
• Au moins un agent édulcorant et/ou agent de goût à un taux jusqu'à 5% en poids et
• Eventuellement un ou plus d'excipients pharmaceutiquement acceptable choisis entre l'agent de stabilisation, diluant, liant, désintégrant, lubrifiant, glissant et surfactant,
dans laquelle ladite composition est dans une forme de dosage d'un comprimé, capsule, comprimé à dissolution rapide ou bien comprimé effervescent.

2. La composition selon la revendication 1, **caractérisé en ce que** la composition pharmaceutique contient du CNA à des doses allant jusqu'à 2500 mg.

3. La composition selon la revendication 1, **caractérisée en ce que**, les édulcorants sont choisis parmi la sucralose, la saccharose, le fructose, le glucose, le galactose, le xylose, la dextrose, le levulose, le lactose, le maltose, la maltodextrine, le mannitol, le maltitol, le maltol, le sorbitol, le xylitol, l'érythritol, le lactitol, l'isomalt, le sirop de mais, la saccharine, les sels de saccharine, l'acésulfame-potassium, l'aspartame, le D-tryptophane, le glyserrisate monoammonique, le néohespéridine dihydrochalcone, la thaumatine, le néotame, l'alitame, le stévioside et le cyclamate.

4. La composition selon la revendication 1, **caractérisé en ce que** les agents aromatisants sont choisis parmi les aromes naturels d'huile (e.g. l'huile de menthe, Partridge l'huile de cassis, l'huile de clou de girofle, l'huile de persil, l'huile d'eucalyptus, l'huile de citron, huile d'orange, etc.), le menthol, menta, l'anéthole, le salicylate de méthyle, l'eucalyptol, la cannelle, l'acétate de 1-méthyle, la sauge, l'eugénol, l'oxanone, l'alpha-irison, la marjolaine, le citron, l'orange, propényle guaethol acétyle, sinnamon, la vanille, le thymol, le linalol, cinnamaldehyde glycérol acétal, p-menthane- 3-carboxamide N-substitué, 3,1- propane diol-1,2 méthoxy.

5. La composition selon la revendication 1, **caractérisé en ce que**, les compositions pharmaceutiques contiennent des agents chélatants tels que l'EDTA, l'acide édétique, l'acide citrique, le citrate de sodium, le citrate de potassium ou les combinaisons de ceux-ci comme des agents stabilisants.

6. La composition selon la revendication 1, **caractérisé en ce que**, la composition pharmaceutique comprend des agents d'alcalinisation choisis parmi les sels de métaux alcalins tels que le carbonate de sodium, de l'hydrogénocarbonate de sodium, l'hydroxyde de sodium, le silicate de sodium, l'orthophosphate d'hydrogène disodique, l'aluminate de sodium; les sels de métaux alcalino-terreux tels que le carbonate de calcium, l'hydroxyde de calcium, le phosphate de calcium dibasique, le phosphate de calcium tribasique, le sulfate de calcium, l'acétate de calcium, le gluconate de calcium, le glycerophosphate de calcium, le carbonate de magnésium, l'hydroxyde de magnésium, le sulfate de magnésium, l'acétate de magnésium, le silicate de magnésium, l'aluminate de magnésium; des composés organiques tels que des amines primaires, secondaires et tertiaires, les amines cycliques, la N, N'-dibenzyletylendiamine, la diéthanolamine, l'éthylènediamine, la meglumine, le glutamate monosodique, le sodium polacryline, l'alginate de sodium comme l'agent de stabilisation.

7. La composition selon les revendications 5 et 6, **caractérisée en ce que** l'agent de stabilisation est de préférence dans la plage de 0-85%, de manière plus préférée 0.1-75% en poids.

8. La composition selon la revendication 1, **caractérisé en ce que**, le diluant est choisi parmi le lactose, la cellulose microcristalline, l'amidon, l'amidon pré-gélatinisé, l'amidon modifié, le phosphate de calcium (dibasique et/ou tribasique), le sulfate de calcium trihydraté, le sulfate de calcium dihydraté, le carbonate de calcium, le kaolin, le lactitol, la cellulose en poudre, le dextrose, les dextrates, la dextrine, le saccharose, le maltose, le fructose, le mannitol, le sorbitol et le xylitol, et est présent de préférence dans la plage de 85%, de manière plus préférée 0.1-75% en poids.

9. La composition selon la revendication 1, **caractérisée en ce que** le liant est choisi parmi l'amidon (e.g. la fécule de pomme de terre, l'amidon de maïs, l'amidon de blé, etc.), le saccharose, le glucose, le dextrose, le lactose, les sucres tels que la maltodextrine, des gommes naturels et synthétiques (telles que la gomme arabique), la gélatine, les dérivés de cellulose (cellulose microcristalline, HPC, HEC, HPMC, la carboxyméthylcellulose, la méthylcellulose, l'éthylcellulose, etc.), la polyvinylpyrrolidone (PVP), le polyéthylène glycol (PEG), des cires, le carbonate de calcium, le phosphate de calcium, des alcools (e.g. le sorbitol, le xylitol, le mannitol) et de l'eau, et est présent de préférence dans la plage de 0-10%, de manière plus préférée 0.1-5% en poids.

10. La composition selon la revendication 1, **caractérisée en ce que**, les désintégrants sont choisi parmi l'amidon (l'amidon de maïs, la fécule de pomme de terre), le glycolate d'amidon sodique, l'amidon pré-gélatinisé, les dérivés de cellulose (e.g., la croscarmellose sodique ou la cellulose microcristalline), la polyvinylpyrrolidone (PVP), la crosspovidone, l'acide alginique, l'alginate de sodium, les argiles (la gomme de xanthane ou la Veegum etc.), des résines d'échange ionique et des systèmes effervescents (les carbonates de métaux alcalins ou alcalino-terreux [carbonate de sodium, le carbonate d'hydrogène de sodium, le carbonate de potassium, l'hydrogénocarbonate de potassium, de calcium carbonate, etc.], des acides solubles dans l'eau polyacides organiques et leurs sels tels que le sulfate d'hydrogène de sodium, de potassium hydrogénosulfate, le dihydrogénophosphate de sodium, l'acide succinique, l'acide tartrique, l'acide adipique, l'acide citrique) et est présent de préférence dans la plage de 0-85%, de manière plus préférée 0.1-75% en poids.

11. La composition selon la revendication 1, **caractérisée en ce que**, le lubrifiant est choisi parmi les stéarates métalliques (le stéarate de magnésium, le stéarate de calcium, le stéarate d'aluminium, etc.), des esters d'acides gras (e.g. le sodium stéaryl fumarate), des acides gras (e.g., l'acide stéarique), des alcools gras, le béhénate de glycéryle, l'huile minérale, des paraffines, des huiles végétales hydrogénées, la leucine, les polyéthylèneglycols (PEG), des sulfates métalliques de lauryle (laurylsulfate de sodium, de magnésium du sulfate de lauryle, etc.), le chlorure de sodium, le benzoate de sodium, l'acétate de sodium et le talc, et est présent de préférence dans la plage de 0-10%, de manière plus préférée 0.1-5% en poids.

12. La composition selon la revendication 1, **caractérisée en ce que**, le glissant est choisi parmi le dioxyde de silicium, le trisilicate de magnésium, la cellulose en poudre, l'amidon, le talc, le phosphate de calcium tribasique, les stéarates métalliques, le silicate de calcium et le sulfate de lauryle métallique, et est présent dans un montant de 1% en poids.

13. La composition selon la revendication 1, **caractérisée en ce que** l'agent de surface est choisi parmi les esters d'acides gras polyoxyéthlene-sorbitane (polysorbates), le laurylsulfate de sodium, le stéarylfumarate de sodium, des éthers alkyliques de polyoxyéthylène, les esters de sorbitan d'acides gras, les polyéthylèneglycols (PEG), dérivés de polyoxyéthylène d'huile de soupçon, le docusate de sodium, de composés d'ammonium quaternaire, les acides aminés tels que la L-leucine, les esters de sucres d'acides gras et les glycérides d'acides gras, et est présent de préférence dans la plage de 0-10%, de manière plus préférée 0.1-5% en poids.

14. La composition selon quelconque des revendications précédentes, **caractérisée en ce que**, la forme galénique comprend une composition pharmaceutique comme ci-dessous ;
CNA à un taux jusqu'à 70% en poids
La vitamine C à un taux jusqu'à 20% en poids
Chlorure de sodium à un taux jusqu'à 5% en poids
L'acide citrique anhydride à un taux de 0-85 % en poids
L'hydrogénocarbonate de sodium à un taux de 0-85 % en poids
La saveur d'aspartame et/ou de citron à un taux jusqu'à 5% en poids
PEG à un taux de 0-10% en poids; et
PVP à un taux de 0-10% en poids.
